(19) Europäisches Patentamt European Patent Office Office européen des brevets

(11) **EP 3 261 568 B1**

(12) **EUROPEAN PATENT SPECIFICATION**

(45) Date of publication and mention
of the grant of the patent:
**17.02.2021 Bulletin 2021/07**

(51) Int Cl.:
*A61B 18/04* *(2006.01)*  *A61B 18/18* *(2006.01)*
*A61F 7/12* *(2006.01)*  *A61N 5/02* *(2006.01)*

(21) Application number: **16755981.4**

(86) International application number:
**PCT/SE2016/050137**

(22) Date of filing: **25.02.2016**

(87) International publication number:
**WO 2016/137382 (01.09.2016 Gazette 2016/35)**

(54) **DEVICE FOR SUPPLY OF HEAT TO BODY TISSUE**

VORRICHTUNG ZUR ZUFUHR VON WÄRME ZU KÖRPERGEWEBE

DISPOSITIF POUR LA FOURNITURE DE CHALEUR AUX TISSUS DE L'ORGANISME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.02.2015 SE 1550217**

(43) Date of publication of application:
**03.01.2018 Bulletin 2018/01**

(73) Proprietor: **Prostalund AB
223 62 Lund (SE)**

(72) Inventors:
• **BOLMSJÖ, Magnus
222 21 Lund (SE)**
• **SCHELIN, Sonny
380 30 Rockneby (SE)**

(74) Representative: **Hansson Thyresson AB
PO Box 73
201 20 Malmö (SE)**

(56) References cited:
| | |
|---|---|
| WO-A1-00/45758 | WO-A1-00/67686 |
| WO-A1-96/36288 | WO-A1-97/01374 |
| WO-A1-99/07315 | WO-A1-99/11190 |
| WO-A1-99/17689 | WO-A1-99/58194 |
| WO-A1-2011/037235 | WO-A2-03/070298 |
| US-A- 5 810 802 | US-A1- 2003 195 595 |
| US-A1- 2003 229 384 | US-A1- 2006 184 163 |
| US-A1- 2008 275 440 | US-A1- 2013 053 619 |
| US-A1- 2014 276 739 | US-B1- 6 312 391 |
| US-B1- 6 445 957 | US-B1- 6 944 504 |

**Description**

TECHNICAL FIELD

The present invention relates to devices for heat treatment of tissue of the living human or animal body. With certain kinds of conditions caused by diseases involving unnatural growths in tissues, treatment with heat gives a good result after treatment. The tissue is heated to the degree that it dies. Examples of disease conditions of this kind are certain types of cancer and benign prostate hyperplasia, BPH. With treatment certain parts of the tissue are heated so that death of the tissue occurs, while other parts of the tissue must or should be protected. The conditions of disease which are primarily focused on here are those which occur in tissue surrounding cavities in the body. As examples, in addition to those mentioned above, cancer of the esophagus, trachea, urethra, and intestine can also be mentioned.

Corresponding conditions of disease can also occur in animals, where similar treatment can be applied. Among these, treatment of domestic animals such as for example dogs, probably would be of most interest,

PRIOR ART

In order to produce heat, different devices can be employed. Laser, microwave, and radio frequency antennas are usually used. A method using the insertion of a container with liquid into the bodily cavity is also known. The liquid expands the container so that good contact against the surrounding tissue is achieved. The liquid is then heated either by supplying warm liquid through a circulating system or by supplying energy to a heating device within the container from which heat is transferred in some way to the liquid and then to the tissue.

Since the volume of the tissue which will be treated varies as well as the ability to absorb heat in this tissue and adjacent tissue, which will not be treated, it is appropriate that continuous monitoring occur during treatment. When treatment is in progress, the heating of tissue occurs. The heating should occur within certain temperature ranges for the best result of the treatment. At too high a temperature unnecessarily great damage occurs in the tissue and at too low a temperature the desired result of treatment does not occur.

It is common that the device for heating incorporates some form of temperature sensor that is arrayed on the element inducing heating in order to monitor the temperature in adjacent tissue. A disadvantage of this design is that the temperature sensor gives information more on the temperature of the element than on the temperature of the tissue.

An example of this type of heating device is shown and described in EP0 370 890. The device encompasses a catheter contained in a microwave antenna that is embodied to emit radio energy to the tissue surrounding the antenna. The catheter is also provided with a cooling channel for cooling the tissue that is located closest to the catheter. In the catheter a temperature transducer is located for reading the temperature of the catheter. The detected temperature is thus not in agreement with the temperature of the tissue that is being treated.

A more developed method of temperature detection is shown and described in WO 96/36288, PCT/SE96/00649. In order to be able to register the rise of temperature directly in the tissue to be treated, a first temperature detection instrument according to PCT/SE96/00649 is connected with a first temperature transducer carrier. The carrier is led through a channel in the catheter and is devised to be extendable through an opening in the catheter. In the opening of the catheter a mechanical guide for the carrier is appropriately aligned in such a manner that the carrier is directed into the tissue at a desired angle in relation to the longitudinal axis of the catheter. The carrier carries three spaced apart temperature transducers. The treatment carried out with the above-mentioned microwave device is often called TUMT (Trans Urethral Microwave Thermotherapy).

Either the carrier or the temperature detection instrument is equipped with a tip that facilitates penetration into the tissue. The temperature detection instrument can be conventionally embodied as a resistive transducer or a semiconductor. The cabling required for transducers of this type is done via channels in the catheter. If a transducer of the optical type is used, a fiber-optic guide is provided through a channel in the catheter US 6 445 957 B1 teaches a device according to the preamble of claim 1.

According to the devices described above and according to other known techniques, the attending physician normally determines the duration and temperature of treatment. In spite of the possibility to conduct continuous temperature detection, there could be problems relating to the positioning of the temperature transducer carrier, and thus of the temperature transducers themselves.

SUMMARY OF THE INVENTION

An object of the invention is to provide a device for supply of heat to body tissue, whereby the disadvantages mentioned above are essentially eliminated. This object is achieved according to the invention by the features indicated in the Patent Claims.

According to the invention there is also the possibility of adjusting the supply of heat beforehand in different ways and to predict the result better. A device according to the invention is used in order to calculate the temperature distribution in the whole prostate based on temperature information from certain intra-prostatic measurement points and based on information on the distribution of energy absorption in the body tissue concerned, when subjected to an energy or heat source. The temperature distribution is determined on the basis of the relation between the temperature in the tissue and absorbed energy,tissue blood flow, i.e. perfusion, and heat conduction. Through continuous measurement of the temperature and the time during which heat is applied, and with continuous monitoring of the temperature distribution in relation to theoretical and/or experimental information on the survival of cells undergoing thermal exposure, the amount of tissue destroyed at certain points in time during the exposure is determined.

In a preferred embodiment the temperature distribution as well as the amount of destructed tissue is continuously presented graphically and/or with the aid of text on a display, so that the attending physician can constantly be informed on current conditions. Heat is supplied to the tissue until a portion of the tissue has been destroyed. The size of the portion can be adjusted. Further advantages and special features of the invention can be seen by the following description, drawings, and dependent patent claims.

BRIEF DESCRIPTION OF THE DRAWINGS

The invention will now be described in more detail with the aid of examples of embodiments with reference to the attached drawings, illustrating embodiments and together with the description, serve to explain the systems. In the drawings

| Fig. 1 | is a right cross sectional view along a median sagittal plane of a patient in supine position showing a prostate gland, a part of the urinary bladder, and a catheter for treatment, |
| Fig. 2 | is a cross sectional view from line II-II in Fig. 1, |
| Fig. 3 | is an enlarged cross sectional view from line III-III of the catheter of Fig. 1, |
| Fig. 4a, 4b and 4c | are diagrams showing temperature curves from different transducers during different treatment cases, and |
| Fig. 5 | is a diagram showing time temperature connection to death of tissue. |

DETAILED DESCRIPTION

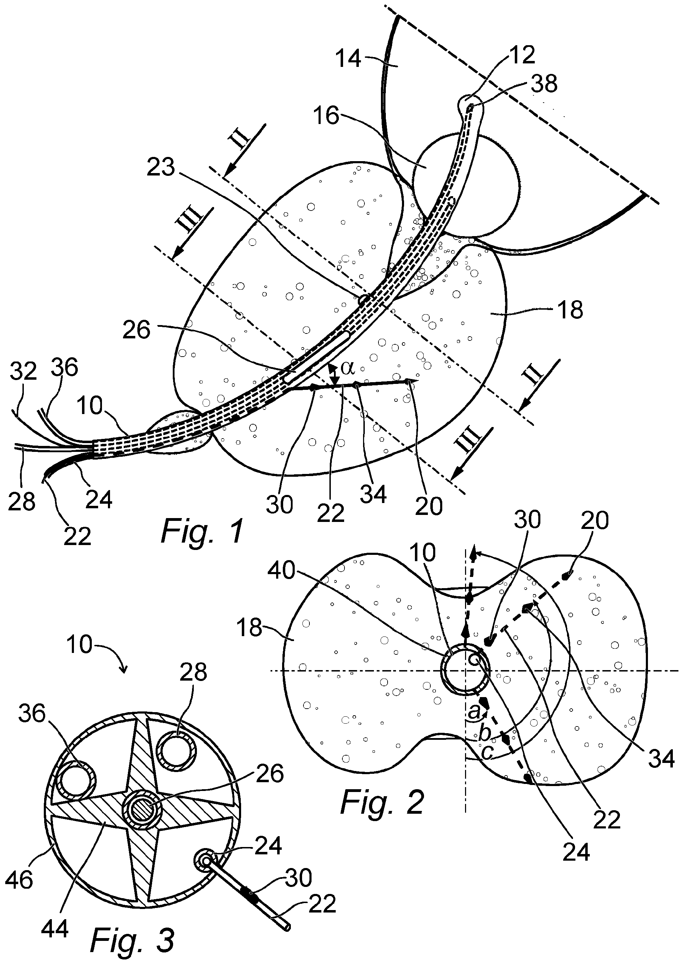

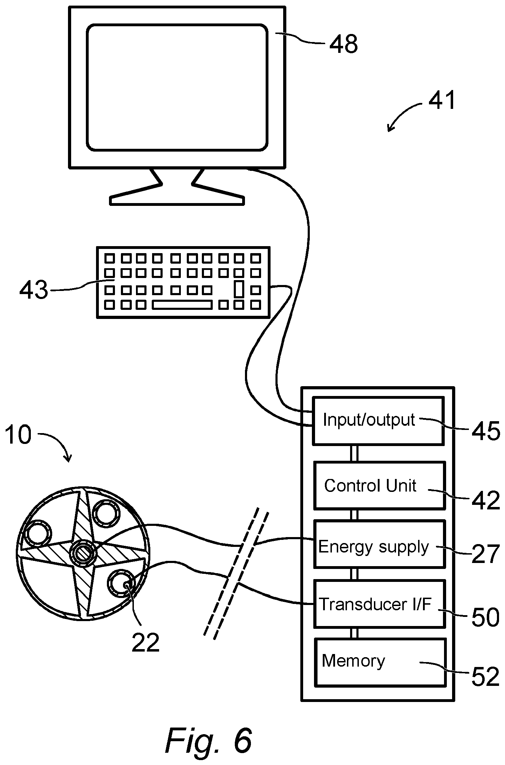

A catheter 10 for the treatment of benign prostate hyperplasia (BPH) is inserted into the urethra according to Fig. 1, so that a tip 12 of the catheter 10 has penetrated into the urinary bladder 14. A balloon 16 connected to the catheter 10 for treatment is expanded inside urinary bladder 14 and prevents inadvertent withdrawal of the catheter 10 for treatment during duration of the procedure that will be described below. An active part, i.e., a part providing energy to the surrounding tissue, of the catheter 10 for treatment is thus arranged within the length of the catheter to be located centrally in the tissue that is to be treated, in this case prostate 18. The catheter for treatment 10 is ductile and flexible in order to be introduced through the urethra to the site of the treatment, yet torsionally rigid to facilitate proper placement of a temperature probe, see below The active part of the catheter 10 is provided with heating means 26 capable of heating body tissue to achieve cell death within a tissue volume (treated area, area of treatment) around the active part of the catheter. In various embodiments the heating means 26 comprises a microwave antenna disposed inside the catheter 10, c.f. Fig. 3. Energy for heating is provided by an energy supply unit 27, c.f. Fig. 6. Energy supply unit 27 and other means and units used during treatment are controlled by a control unit 42, c.f. Fig. 6 and corresponding description below.

In order to be able to follow the temperature development in the tissue during heat treatment a first temperature transducer 20 is located on a carrier 22 also known as a probe 22. Carrier 22 is designed to be advanced through a channel or conduit 24 (c.f. Fig. 3), which runs through the catheter for treatment at least from a base end intended to stay outside the patient's body to a position where conduit is provided with means to let probe advance out of catheter and into surrounding tissue. Carrier 22 or temperature transducer 20 of carrier 22 is preferably provided with, or as, a tip that can penetrate in part a membrane or wall the catheter 10 for treatment and in part the tissue. The membrane or wall so intended to be penetrated is preferably arranged close to the active part of the catheter 10. The membrane has the advantage to keep catheter sealed until probe is advanced into tissue. Conduit 24 is embodied so that carrier 22 with temperature transducer 20 is advanced out of the catheter for treatment at a suitable angle and can be extended to a suitable radial distance from the catheter for treatment. It is also possible to provide a specially angled instrument in the terminal section of conduit 24 in order to achieve the desired angling of carrier 22 and temperature transducer 20.

The carrier 22 of the catheter 10 for treatment can also be provided with a second temperature transducer 30, arranged a first distance from the tip of the carrier. The first distance is chosen such that when the carrier is advanced to the satisfaction of the treating physician, the second temperature transducer becomes located just outside the catheter. Information on the temperature close to catheter 10 for treatment is thus obtained from the second temperature transducer 30. In the embodiment shown in Fig. 1-Fig. 3 the device according to the invention is also provided with a third temperature transducer 34. Third temperature transducer 34 is preferably also located on carrier 22 between first temperature transducer 20 and second temperature transducer 30, so that the temperatures can continuously be determined at specified distances from catheter 10 for treatment.

A liquid channel 28 is also incorporated in the catheter for treatment. It opens into balloon 16 and through it liquid can be directed for expansion of balloon 16 when the catheter for treatment is in place. Liquid channel 28 is also used to empty balloon 16 at the conclusion of treatment and before the catheter for treatment is withdrawn from the urethra. A conventional syringe or similar is preferably used for insertion of liquid and for emptying balloon 16.

A feed cable 32, by means of which heating means 26 is supplied with energy, is also, as a side effect, warmed as a result of energy loss within the feed cable. In order to avoid injury of tissue beyond the area of treatment, for example, on the sphincter that surrounds the urethra outside the prostate, feed cable 32 is cooled. This is done by providing cooling channels (not shown) in catheter for treatment 10, preferably around feed cable 32. In an embodiment according to the invention the cooling channels have a limiting wall at which the cooling liquid circulating in the cooling channels returns. In this manner cooling of heating means 26 itself is avoided, which in turn means that the power, which needs to be supplied from an energy supply unit 27, can be less. With lower levels of power the risk of injury to healthy tissue is reduced.

At high temperatures in the range of 90-150°C the tissue also hardens and forms a crust. The crust can prevent or lessen problems that can occur if the prostate gland swells in connection with the treatment. During treatment a high temperature is reached in tissue closest to catheter 10 and the portion of the urethra that passes through the prostate at the area of treatment will be affected to a high degree and injured. This portion of the urethra, however, restores itself relatively quickly.

In a preferred embodiment heating means 26 comprises a microwave antenna. Since the urethra is completely filled by catheter 10 in the area of treatment and no free space remains, the fit of the microwave antenna against the tissue will also be very good. The adjustment of impedance between the antenna and the tissue is very good, which simplifies dimensioning of the antenna and energy supply unit and facilitates the setting of microwave power.

When treatment is finished, the energy supply to heating means 26 is terminated. It is not suitable to remove the catheter for treatment as long as any part of the catheter has a temperature such that injuries could occur with the passage of the catheter through the body. Cooling water supply is maintained and when it is judged that catheter can be removed without risk, the carrier or probe 22 is first retracted into catheter, and then catheter may be removed from patient.

In cases of treatment involving the prostate or urine bladder, whereby catheter 10 having a rounded tip is introduced into urine bladder 14, drainage of urine and possibly of other liquid from the urine bladder can occur through a drainage channel 36 provided in catheter 10. The drainage channel runs through the full length of catheter 10 and ends with opening 38 near the tip of catheter 10. With certain types of treatment, it can be suitable to leave catheter 10 in place for a certain time after treatment. It is the function of the drainage channel to drain the urine bladder even during this time.

Rotational orientation of the catheter 10 is important for extending and positioning the carrier 22 in a favorable direction. An orientation meter 23 is arranged on the catheter 10 for obtaining information about the present orientation of the catheter. Suitably there is provided a visual marking on the outer surface of the catheter and extending in the longitudinal direction of the catheter to show to the treating physician in what direction the carrier or probe 22 will be deployed. Together with the torsional rigidity of the catheter the marking will provide information to physician. However, since effect of torsional rigidity depends on catheter material and length of catheter there could be differences between estimated and actual position of probe 22. An improvement in this regard is the provision of the orientation meter 23, which preferably senses the orientation of the catheter relative to an absolute reference such as the gravitational force. The orientation meter 23 may be embodied as a 3-axis accelerometer and/or a 3-axis gyroscope. Preferably the orientation meter is connected to an indicator or display using thin wires extending through the catheter. The orientation meter is arranged with its reference direction being zero or a known offset value relative to opening in catheter for probe or carrier 22.

The treated and dead tissue is reabsorbed or rejected and eliminated with urine. A cavity in the prostate caused by the removal of tissue ensures the unconstructed correct passage of urine. The cavity at first has a shape that corresponds to the volume heated the most during treatment.

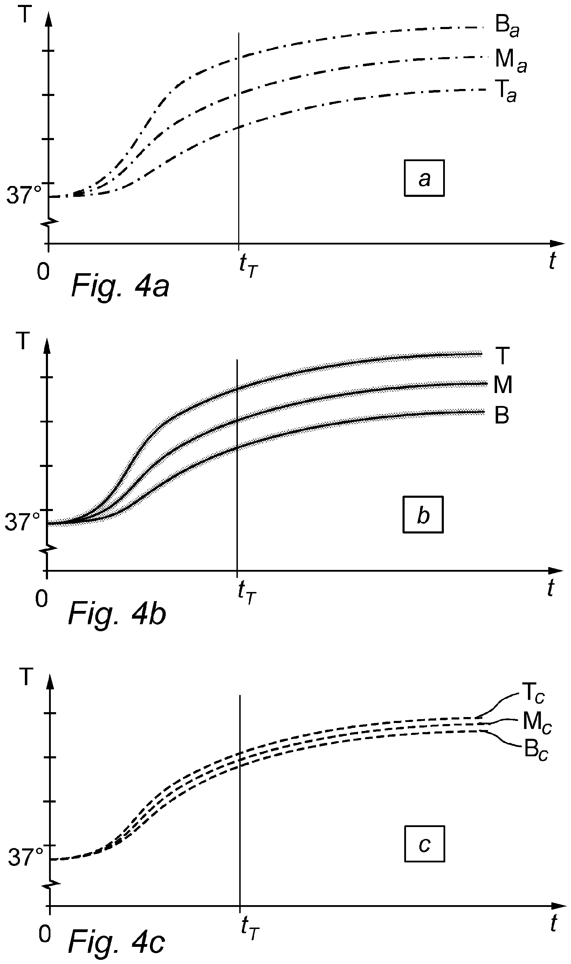

As shown in Fig. 2 the carrier 22 is fully extended from the catheter 10 in order to position said first temperature transducer 20, said second temperature transducer 30 and said third temperature transducer 34 for measuring the temperature development during treatment. The probe or carrier 22 preferably should be extended at an angle depicted at b in a plane transverse to the prostatic urethra. This plane is in general parallel to direction of the gravitational force when patient is lying on his back. At this angle b all three temperature transducers are correctly positioned in the prostate tissue. The angle b is preferably in the interval of 30-60 degrees or in the interval of 210 to 240 degrees. Temperature information from all temperature transducers will provide basis for a correct evaluation of the treatment process. A correctly positioned probe or carrier 22 with temperature transducers will give a set of temperature curves as shown in Fig. 4b with unbroken lines. Catheter 10 extends inside the urethra 40.

One difficulty that may arise is that both angle a (approximately 30 degrees) and *c* (approximately 180 degrees) of probe or carrier 22 as shown in Fig. 2 will produce temperature readings that may confuse the treating physician, if he or she is not aware of if the actual angle is one of type "a", "b", or "c", and thus non-desired heating of prostate tissue and other body tissues surrounding the prostate may result. Now turning to figures 4a, 4b, and 4c, which are diagrams showing temperature curves from different transducers during different treatment cases. More specifically, at angles c the temperature profiles would be pretty the same for tip (T), mid (M) and base (B) temperature transduce curves, as indicated by curves $T_c$, $M_c$, and $B_c$ in figure 4c. If the angle is small, as denoted "a", the temperature profile of tip (T) will be the lowest and the temperature profile of base (B) the highest, i.e., the aggregated temperature profile will be the opposite of what would be desired, see $B_a$, $M_a$ and $T_a$ temperature curves of fig. ¤4a. The reason for this is the anatomical conditions in the area. The curvature of urethra gives rise to this effect. The urethra curves with a concave shape towards the front/abdominal side of the body, and convex shape towards the back side of the body. Because the urethra curves towards abdominal side or "upwards" as closer to the bladder it gets, the tip of the temperature probe 22 will reach at an approximately 3 o'clock position, seen from a plane closest to the balloon. The temperature probe is normally deployed from the catheter approximately 30 mm behind the balloon. In the case that the temperature probe 22 exits at 12 o'clock the curvature of the urethra will cause the tip 20 to end up close to the urethra and cause the temperature curves of T, M, and B to be approximately the same. If, on the other hand, the temperature probe 22 instead exits at approximately 6 o'clock (angle "a"), the tip of temperature probe 22 would end up a much longer distance from the urethra, even outside the reach of the microwaves, which will result in temperature curve from tip (T) temperature transducer 20 being the lowest i.e. the coldest. See figure 4a curve $T_a$.

Such angles as "a" and "c" therefore should, if possible, be avoided. Temperature curves of Fig. 4a, 4b, and 4c indicate how temperature will develop with these angle settings of temperature probe/carrier 22. Some further explanation of the temperature curves is set out below with reference to these figures.

Note that the angle b in the transversal plane as defined above is not to be confused with an angle $\alpha$ (alfa) between the longitudinal direction of the catheter and the portion of the carrier 22 or probe 22 extending outside the catheter 10. The opening for the probe 22 guides the probe in a fixed direction producing an angle alfa of preferable around 45 degrees.

The cross sectional view of Fig. 3 shows catheter 10 from line III-III in Fig. 1. Four wings 44 extend radially between a central core and an outer wall 46 to provide stability. Feeding cable 32 extends through a cavity in the central core and the heating means 26 is arranged in the cavity. Liquid channel 28 extends through a first cavity formed between two wings 44 and the outer wall 46, and drainage channel 36 extends through a second cavity formed between two wings 44 and the outer wall 46. The cavities can be used for circulating a cooling liquid through the catheter 10.

Conduit 24 extends through a third cavity formed between two wings 44 and the outer wall 46. A section of the carrier 22 with second temperature transducer 30 extends out through an opening in the outer wall 46 of catheter 10. Opening in the outer wall may initially be covered with penetrable membrane as disclosed above. In various embodiments different combinations of drainage channel 36, liquid channel 28 and conduit 24 are arranged in other cavities.

When heating means 26 is activated and produces heat to surrounding tissues the temperature will increase. A correct positioning and orientation of catheter 10, carrier 22 supporting said first temperature transducer 20, said second temperature transducer 30 and said third temperature transducer 34 will produce a desired increase of temperature in the surrounding tissue. In Fig. 4b curve T (tip) indicates temperatures obtained from first temperature transducer 20, curve B (base) temperatures obtained from second temperature transducer 30, and curve M (mid) temperatures obtained from third temperature transducer 34, during such correct positioning case.. Some variations at each location from the desired temperature development are normal and are indicated by wider gray lines.

However, should catheter 10 be rotated in either direction from a correct orientation "b" as indicated in Fig. 2 actual temperatures measured by the temperature transducers will form other curves, or rather curves with exchanged position relative to each other, as indicated in figures 4a, and 4c. If actual temperature curves deviate from the expected curves outside normal variations as defined by the wider gray lines indication will be given by control unit 42 via display unit 48. At such indication treatment can be interrupted automatically or by a manual step performed by responsible personnel. It is possible also to proceed with the treatment until a certain total amount of energy has been supplied to the heating means 26 as indicated by the control unit 42.

Different conditions such as supply of blood in different parts of the tissue will have an impact on the temperature development. However, should the temperature after a treatment period of $t_T$ deviate more than within an allowed interval, an indication of error or possible error is given.

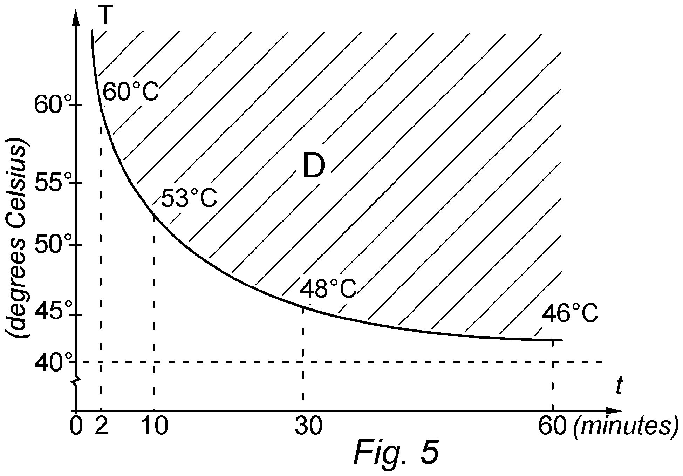

The diagram of Fig. 5 illustrates cell kill or cell destruction in relation to time t (minutes) and temperature T (degrees Celsius). The area of cell destruction depicted by D indicates the combination of time and temperature that will lead to death of tissue. The purpose of the treatment is to reach the area D for prostate tissue surrounding the heating means 26. It can be seen that at lower temperatures close to 40 degrees Celsius cell death will not occur despite long exposure time. At temperatures of 60 degrees Celsius and higher, cell death will occur after only a few minutes.

The block diagram in Fig. 6 shows schematically different functional blocks that can be included in a treatment assembly 41 with a catheter 10 for treatment according to the invention. As indicated above, heating means 26 is supplied with energy from an energy supply unit 27. A central control unit 42 is operatively connected to energy supply unit 27 and to an input/output means 45. Exterior units such as a display unit 48 and a keyboard 43 are operatively connected to said input/output means 45. Control unit 42, keyboard 43, and display unit 48 can also be included in a conventional computer with monitor and keyboard.

Said first temperature transducer 20, said second temperature transducer 30 and said third temperature transducer 34 all supported by the carrier 22 are operatively connected to a transducer interface 50. Temperature data from said temperature transducers are received in said transducer interface 50. Calculations, comparisons and control steps can be performed in said control unit 42 or said transducer interface 50 that is operatively connected thereto. A memory or memory unit 52 is operatively connected to the transducer interface 50, said input/output means 45 and said energy supply unit 27 to hold and store data relating to temperature, treatment process and physiological conditions. In various embodiments the orientation meter 23 is connected to the transducer interface to relay information regarding rotational angle of the catheter 10 to the display unit 48. The information on such rotational angle may also be transferred to the control unit 42 for use as a further basis for calculation of control signals to energy supply unit 27.

For treatment with TUMT and similar methods of treatment the temperature of the tissue is determined to the greatest degree by three different processes: *i)* generation of heat by absorption of microwave energy or another source of radiated energy, *ii)* heat distribution as a result of heat conduction in the tissue, and *iii)* heat loss as a result of the flow of blood (tissue perfusion). Parameter *i)* is determined by the current catheter for treatment being used, *ii)* can be calculated, while *iii)* is dependent on the patient and is unknown. The relationship is given by a known equation for bio-heat:

$$\rho c \frac{dT}{dt} = \lambda \Delta T - \varpi_b \rho_b c_b \rho (T - T_a) + Q_s + Q_m \qquad \text{(Equation 1)},$$

where $\rho$ (rho) (kg m$^{-3}$) is the density of the prostate, c (J kg$^{-1}$K$^{-1}$) is the specific heat capacity of the prostate, T (°C) is the temperature of the prostate at the time t (seconds), $\lambda$ (W m$^{-1}$ K$^1$) is the heat conduction in the prostate, $\Delta$ is the Laplace operator, $\varpi_b$ is the perfusion of the tissue (m$^3$ kg$^{-1}$ s$^{-1}$), $\rho_b$ (kg m$^{-3}$) is the density of the blood, $c_b$ (J kg$^{-1}$ K$^{-1}$) is the specific heat capacity of the blood, $T_a$ is the arterial temperature (°C), $Q_s$ (W m$^{-3}$) is generation of heat as a result of microwave absorption, and $Q_m$ (W m$^{-3}$) is the generation of heat by metabolism.

The metabolic term $Q_m$ can be ignored in the context of heat treatment. The thermal characteristics of the prostate tissue have been calculated from its water content, which is assumed to be 80%. The equation or corresponding data can be stored in memory unit 52, so that it can be solved continuously during the application of heat.

Since it can be assumed that microwave absorption is symmetrical in the radial orientation, cylindrical geometry was used for the numerical solution of equation 1. The finite differential technique was used for the solution. The microwave absorption term $Q_s$ in equation 1 was determined by the specific absorption rate (SAR, W/kg) in a certain catheter for treatment 10. A practical way to determine the specific rate of absorption is to place a TUMT catheter in a tissue-like body, for example made of the material known as TX-150, and measure the temperature distribution in the body after heating with a microwave output of 50W for 60 seconds. One way to measure the specific absorption rate SAR is described in detail in the British Journal of Urology 78 (1996), pages 564-572.

In memory unit 52 known data as to the survival of cells at different cell temperatures during different durations of treatment are stored, for example in the form of suitable equations, data tables, or similar. Memory unit 52 is suitably configured so that stored data is complemented and, if necessary, corrected as new results of treatments are collected. Tissue damage caused by the treatment can be described mathematically by an Arrhenius equation according to the following:

$$\Omega = A \int e^{-E_a/(RT)} dt \, , \qquad \text{(Equation 2)}$$

where $\Omega$ (omega) is the degree of accumulated damage during the time of treatment t, A is the Arrhenius constant (3.1$10^{98}$ s$^{-1}$), $E_a$ is the energy of activation of the cells, (6.3$10^5$ J mol$^{-1}$), R (J mol$^{-1}$K$^{-1}$) is the universal gas constant, and T (K) is the absolute temperature of the tissue. The tissue is assumed to be destroyed at $\Omega \geq 1$.

A suitable measure is that patient and result of treatment are followed up at certain intervals after treatment, for example, every month for a certain period. In memory unit 52, there are stored data on blood flow and on other factors that affect heat absorption and heat dispersion in the type of tissue undergoing treatment. With exact data it is possible to store information in memory unit 52 which enables creation of a model of the tissue with respect to the factors cited above that models heat distribution very close to the actual tissue. Preferably, these later data are continually complemented and corrected even after the treatment.

Before heat treatment of a patients prostate certain current physical factors are determined, for example, the size of the prostate, the degree of narrowing ofi prostatic urethra, and the distance between the prostate and the rectum. Such physical factors or conditions may be determined with the aid of an ultrasound examination. With information on these conditions a suitable type of catheter for treatment and suitable temperature for treatment are determined. Different types of catheters may differ in diameter, in overall length, in length and power of heating means 26, and/or in distance from heating means 26 to inflatable balloon 16. Instead of duration of treatment, which according to previous devices was a decisive factor, a weight value and/or a volume value is determined for the desired volume/weight of the tissue that is to be treated, so that it can be destroyed. The value is given so that it is available to control unit 42, for example, by feeding it via a keyboard 43 and input/output unit 45. A highest value for temperature, or mean value for the temperature of the treatment, or a temperature range are preferably also input. The device can also be provided with further memories for storage of suitable combinations of volume/weight and temperature of the treatment. In the latter case, a norm value is automatically selected for the temperature of the treatment.

Also orientation meter 23 is operatively connected to said control unit 42 With these inputs mentioned above, the control unit 42 can calculate and send a control signal to energy supply unit 27, which supplies energy to the heating means so that heating means 26 begins to emit energy for heating of the tissue. Energy supply unit preferably supplies electrical energy while heating means preferably emit microwave energy. First temperature transducer 20 and preferably also second temperature transducer 30 and third temperature transducer 34 provide continuous information regarding the current temperature of the surrounding tissue, both near heating means 26 and at a certain distance from it in the tissue under treatment, as disclosed above

Control unit 42 is operatively connected to temperature transducer 20 and preferably also to transducers 30 and 34 and can, depending on the current temperature in the area of treatment, govern energy supply unit 42, so that suitable output is directed to heating means 26. By this means, it is possible to raise the temperature strongly in the surrounding tissue, so that death of the tissue occurs in the desired way. Data on temperature from temperature transducers 20, 30 and 34 can also be shown continuously in display unit 48. With the aid of input/output means 45 it is also possible to indicate a suitable level of output power from heating means 26, or as it can be rated from energy supply unit 27. The output level affects the temperature that the tissue obtains and may in certain cases affect the level of pain experienced by the patient.

Control unit 42 is configured to, during treatment, to continuously compare data from temperature transducers 20, 30, and 34 with the data stored in memory unit 52, and control unit 42 can by this means continuously calculate how the temperature varies in the prostate tissue at various distances from catheter for treatment 10. It is also possible in this way for control unit 42 continuously to calculate the volume/weight of the tissue that has been treated in the desired manner and where the desired result of treatment (cell death) has been achieved in the tissue.

Control unit 42 is also configured such that information calculated in the above way is continuously sent to display 48, so that besides numerical data regarding the treated volume/weight, the attending physician has an image of how the treatment is proceeding. A suitable method is that a schematic image similar to that in FIG 1 is shown on the display 48. Data, in particular image data, regarding the prostate that will be treated are suitably collected beforehand, for example, by means of obtaining at least one beforehand image over the organ to be treated by means of e.g. x-ray imaging, ultrasound imaging, or MRI, so that the image which is shown on display unit 48 is in agreement with actual conditions. An image corresponding to the catheter 10 for treatment that will be used is preferably superimposed over such beforehand image of the tissue.

The temperature in different parts of the prostate tissue is continuously calculated by the control unit 42 on the basis of the measured temperatures, and the current temperature is shown in the image of the prostate, for example, by means of markings in different colors. At the same time different graphs and/or tables regarding temperatures, blood flows, and volume/weight of the tissue under treatment are shown. The attending physician can in this way follow the treatment and continuously receive indications on how much tissue has been treated and where in the prostate tissue death has occurred.

A timer (not shown), operatively connected with control unit 42, or being an integral part of the same, continuously sends information on the time, so that the measured data becomes related to the treatment timeline. When one of the values for the volume or weight of the tissue being treated, as calculated by control unit 42, is in agreement with the preset value, the treatment can automatically be interrupted. With the aid of the display unit 48 or another indication instrument it is possible instead to indicate when the value set has been reached, so that the attending physician can interrupt the supply of heat manually. Corresponding interruption or indication can also occur if the temperature measured or calculated in some part of the tissue exceeds a threshold value, or if other input data, such as, for example, the temperature in the rectum or in the bladder indicates a risk for the patient. In such embodiments means for measuring rectal and bladder temperature are arranged to be input to the transducer interface 50 or input/output unit 45.

It is also possible continuously to alter certain settings during treatment, for example the desired temperature of treatment or the microwave input, without affecting or needing to change the value set for volume/weight. Instead the duration of treatment is affected. The duration of treatment can vary quite significantly with the method described above, depending on physiological differences and does not control the treatment in any decisive manner in comparison with methods of treatment employed previously.

Below two particular control methods will be described in more detail. The control methods are called "A" and "B" respectively. The purpose of both methods is to achieve cell-death of the desired amount of tissue in spite of unintentional undesired placement of temperature probe 22.

The control method A comprises the following steps:

- Reading of temperature values of temperature transducers T (tip), M (mid) and B (base);
- Comparing temperature values;
- Based on comparison, deciding whether temperature values of T and M are both higher than temperature value of B during full length of treatment or not;
- Using eq. 1 to estimate the perfusion $\varpi_b$.
- Based on experience, deciding whether perfusion $\varpi_b$ is low or not low;
- Based on the preceding steps, if temperature values of T and M are both higher than temperature value of B during full length of treatment, and perfusion $\varpi_b$ is low then the treatment is based on cell destruction (cell kill) as estimated using eq. 2, and supply of heat is terminated when a certain percentage of destruction is achieved, preferably around 20% of the total volume of the prostate gland volume.

Clinical studies have shown that under these conditions the cell kill estimation algorithm performs excellent. As a rule intended cell kill (approx. 20 %) is reached within 7 to 15 minutes of treatment. An end temperature is in most of these cases within interval of 50 - 70 degrees Celsius for temperature values of T and M and lower for B. The "Endpoint", i.e. the criteria defining termination of treatment is in this case the reaching of estimated desired cell kill, typically 20% of size of prostate gland before treatment.

The control method B comprises the following steps:

- Reading of temperature values of temperature transducers T (tip), M (mid) and B (base);
- Comparing temperature values;
- Based on comparison, deciding whether temperature values of T and M are both higher than temperature value of B during full length of treatment or not;
- Using eq. 1 to estimate the perfusion $\varpi_b$.
- Based on experience, deciding whether perfusion $\varpi_b$ is low or not low;
- Based on the preceding steps, if temperature values of T and M are both not higher than temperature value of B during full length of treatment, or perfusion $\varpi_b$ is not considered low then the treatment is based on amount of microwave energy used during treatment, supply of heat is terminated when the monitored amount of supplied microwave energy has reached a preset amount E, preferably E is set based on weight of prostate gland before treatment to E=0.5*m, where E is microwave energy in kJ and m is weight of prostate gland in gram.

Example: If weight of prostate gland is 50 gram the treatment should be terminated after 25 kJ. In order to retain the highest degree of safety a limit is set at which treatment is terminated, in spite of a big gland, and this limit may as a suggestion be 50 kJ.

The inventive idea and rationale for using the point in time when microwave energy supplied to the tissue exceeds a certain amount has been derived from eq. 1; if you eliminate perfusion, the temperature of the prostate during treatment will only depend on supplied energy. The perfusion blood flow can easily be cut during treatment by, before treatment injecting adrenaline into the prostate, which will cause the blood vessels to constrict temporarily, lowering the perfusion of the gland to almost naught. As a secondary endpoint of treatment, it is therefore possible to use a level of total amount of supplied energy (kJ).

Further, temperatures exceeding predetermined values in any tissue outside of the treated area may be indicated by sounding of an alarm signal. Temperatures exceeding predetermined values in the tissue outside of the treated area may cause the control unit 42 to automatically interrupt the supply of heat.

It will be apparent to those skilled in the art that various modifications and variations can be made without departing from the inventive concept. Other embodiments will be apparent to those skilled in the art from consideration of the specification and practice disclosed herein. It is intended that the specification and examples be considered as exemplary only.

**Claims**

1.  A device for supply of heat to body tissue, comprising

    - a catheter (10) having a tip (12) insertable into a human bladder and supporting heating means (26),
    - an elongated carrier (22) supporting spaced apart a first temperature transducer (20), a second temperature transducer (30) and a third temperature transducer (34), said elongated carrier (22) being extendable from the catheter in the vicinity of said heating means (26) into body tissue,
    - a control unit;
    - a memory means,

    wherein
    the control unit (42) is operatively connected to the memory means (52) for storage of heating data corresponding to the survival of cells as a function of cell temperature and time, and also operatively connected to said first temperature transducer (20), said second temperature transducer 30) and said third temperature transducer (34), said control unit (42) is configured to obtain a first set of temperature data from said first temperature transducer (20), said second temperature transducer (30) and said third temperature transducer (34), and
    said control unit is configured to indicate differences between temperatures of said first, second, and/or third transducers, **characterized in that** the device further comprises an orientation meter (23) for sensing an orientation of the catheter (10)

2.  The device according to Claim 1, **characterized in that** said control unit (42) is operatively connected to said memory means (52) for storage of a second set of temperature data corresponding to estimated temperature changes in body tissue at different distances from said catheter, and to compare said first set of temperature data to said second set of temperature data.

3.  The device according to Claim 1 or 2, **characterized in that** differences between simultaneous temperature values of first transducer (20) and second transducer (30) are stored and displayed.

4.  The device according to Claim 3, **characterized in that** differences between simultaneous temperature values of third transducer (20) and second transducer (30) are stored and displayed.

5.  The device according to Claim 1 or 2, **characterized in that** said control unit (42) is operatively connected to time-measuring means for continuous determination of the point in time when the tissue located at a certain distance from heating device (10) has reached a certain temperature.

6.  The device according to Claim 1 or 2, **characterized in that** memory means (52) is designed to store dissipation data from a certain body tissue that pertain to the tissue's ability to dissipate heat.

7.  The device according to Claim 1 or 2, **characterized in that** said first temperature transducer (20) is designed to penetrate and to be inserted into the tissue to which heat is to be supplied.

8.  The device according to Claim 1 or 2, **characterized in that** the second temperature transducer (30) is provided in the vicinity of heating means (26) for measuring the temperature in the tissue close to catheter (10) for treatment.

9.  The device according to Claim 8, **characterized in that** the third temperature transducer (34) is designed to penetrate and be inserted into the tissue to which heat is to be supplied, to a distance from said catheter (10) for treatment that is between the first (20) and the second (30) temperature transducer.

**10.** The device according to Claim 1 or 2, **characterized in that** the heating means (26) is a microwave antenna (26) for heating the surrounding tissue.

**11.** The device according to Claim 3, **characterized** in that control unit (42) is operatively connected to a display unit (33) for presentation of the temperature in different parts of the tissue under treatment, and that said control unit (42) is configured to determine the temperature to be presented on the basis of signals from said first temperature transducer (20) and through calculation on the basis of how the tissue temperature is changed over time in the tissue in which the first temperature transducer (20) is inserted.

**12.** The device according to any of the previous claims, wherein the control unit (42) is configured to be able to execute two different automatic methods for determining when to terminate energy supply to heating means (26).

**13.** The device according to claim 12 wherein one automatic method for determining when to terminate energy supply to heating means (26) is based on estimation of amount of cell death based on measured temperatures and duration of time since onset of energy supply to heating means (26).

**14.** The device according to claim 12 wherein one automatic method for determining when to terminate energy supply to heating means (26) is based on an amount of energy supplied to the heating means (26) since onset of energy supply to heating means (26).

**15.** The device according to claim 12 wherein the two automatic methods for determining when to terminate energy supply to heating means (26) are the ones of claim 13 and 14 respectively.

**16.** The device according to any of claims 13, 14 or 15, wherein the control unit (42) is configured to make a choice of which one of the two different automatic methods to use for determining when to terminate energy supply to heating means (26).

**17.** The device of claim 16, wherein the control unit (42) is configured to make the choice of method based on differences between simultaneous temperature values of first transducer (20) and second transducer (30) or, as an alternative, based on differences between simultaneous temperature values of third transducer (34) and second transducer (30), or based on a combination thereof.

**18.** The device for supply of heat to body tissue according to claim 1, further comprising that said control unit is configured (42) to calculate the amount of destructed tissue with the aid of eq. 1 supported by eq. 2 or similar equations, and wherein eq. 1 reads:

$$\rho c \frac{dT}{dt} = \lambda \Delta T - \varpi_b \rho_b c_b \rho (T - T_a) + Q_s + Q_m$$

wherein $\rho$ (rho) (kg m-3) is the density of the prostate, c (J kg-1K-1) is the specific heat capacity of the prostate, T (°C) is the temperature of the prostate at the time t (seconds), $\lambda$ (W m-1 K-1) is the heat conduction in the prostate, $\Delta$ is the Laplace operator, $\varpi_b$ is the perfusion of the tissue (m3 kg-1 s-1), $\rho_b$ (kg m-3) is the density of the blood, $c_b$ (J kg-1 K-1) is the specific heat capacity of the blood, $T_a$ is the arterial temperature (°C), Qs (W m-3) is generation of heat as a result of microwave absorption, and Qm (W m-3) is the generation of heat by metabolism, and wherein eq. 2 reads:

$$\Omega = A \int e^{-E_a /(RT)} dt$$

wherein $\Omega$ (omega) is the degree of accumulated damage during the time of treatment t, A is the Arrhenius constant $(3.110^{98}$ s-1), $E_a$ is the energy of activation of the cells, $(6.310^5$ J mol-1), R (J mol-1K-1) is the universal gas constant, and T (K) is the absolute temperature of the tissue, the tissue being assumed to be destroyed at $\Omega \geq 1$.

19. The device for supply of heat to body tissue according to any of the preceding claims wherein the orientation meter (23) senses the orientation of the catheter relative to an absolute reference..

20. The device for supply of heat to body tissue according to claim 19 wherein the orientation meter (23) senses the orientation of the catheter relative to the gravitational force.

21. The device for supply of heat to body tissue according to claim 19 or 20 wherein the orientation meter (23) is embodied as a 3-axis accelerometer and/or a 3-axis gyroscope.

22. The device for supply of heat to body tissue according to claim 21 wherein the orientation meter (23) is embodied as a 3-axis accelerometer.

23. The device for supply of heat to body tissue according to claim 21 wherein the orientation meter (23) is embodied as a 3-axis gyroscope.

24. The device for supply of heat to body tissue according to any of claims 19-23
wherein the orientation meter (23) is connected to an indicator or display using thin wires extending through the cateheter.

25. The device for supply of heat to body tissue according to any of claims 19-24
wherein the orientation meter (23) is arranged with its reference direction being zero or a known offset value relative to opening in catheter for probe or carrier (22).

26. The device for supply of heat to body tissue according to any of claims 19-25
wherein orientation meter (23) is operatively connected to said control unit (42).

27. The device for supply of heat to body tissue according to claim 26 wherein the control unit (42) is configured to calculate and send a control signal to the energy supply unit (27), which supplies energy to the heating means so that heating means 26 begins to emit energy for heating of the tissue, based on inputs from said orientation meter (23).

**Patentansprüche**

1. Vorrichtung für eine Zufuhr von Wärme zu Körpergewebe, umfassend:

   - einen Katheter (10), der eine Spitze (12) aufweist, die in eine menschliche Blase einführbar ist und ein Heizmittel (26) trägt,
   - einen länglichen Träger (22), der beabstandet einen ersten Temperaturfühler (20), einen zweiten Temperaturfühler (30) und einen dritten Temperaturfühler (34) trägt, wobei der längliche Träger (22) von dem Katheter in der Nähe des Heizmittels (26) in das Körpergewebe ausziehbar ist,
   - eine Steuereinheit;
   - ein Speichermittel,

   wobei die Steuereinheit (42) mit dem Speichermittel (52) für eine Speicherung von Heizdaten wirkverbunden ist, die dem Überleben von Zellen als Funktion von einer Zelltemperatur und einer Zeit entsprechen, und auch mit dem ersten Temperaturfühler (20), dem zweiten Temperaturfühler (30) und dem dritten Temperaturfühler (34) wirkverbunden ist, wobei die Steuereinheit (42) konfiguriert ist, um einen ersten Satz von Temperaturdaten von dem ersten Temperaturfühler (20), dem zweiten Temperaturfühler (30) und dem dritten Temperaturfühler (34) zu erhalten, und die Steuereinheit konfiguriert ist, um Unterschiede zwischen den Temperaturen des ersten, des zweiten und/oder des dritten Fühlers anzuzeigen, **dadurch gekennzeichnet, dass** die Vorrichtung ferner einen Ausrichtungsmesser (23) zum Erfassen einer Ausrichtung des Katheters (10) umfasst.

2. Vorrichtung nach Anspruch 1, **dadurch gekennzeichnet, dass** die Steuereinheit (42) mit dem Speichermittel (52) für die Speicherung eines zweiten Satzes von Temperaturdaten wirkverbunden ist, die geschätzten Temperaturänderungen in dem Körpergewebe in unterschiedlichen Abständen von dem Katheter entsprechen, und um den ersten Satz von Temperaturdaten mit dem zweiten Satz von Temperaturdaten zu vergleichen.

3. Vorrichtung nach Anspruch 1 oder 2, **dadurch gekennzeichnet, dass** Unterschiede zwischen gleichzeitigen Temperaturwerten des ersten Fühlers (20) und des zweiten Fühlers (30) gespeichert und angezeigt werden.

4. Vorrichtung nach Anspruch 3, **dadurch gekennzeichnet, dass** Unterschiede zwischen gleichzeitigen Temperaturwerten des dritten Fühlers (20) und des zweiten Fühlers (30) gespeichert und angezeigt werden.

5. Vorrichtung nach Anspruch 1 oder 2, **dadurch gekennzeichnet, dass** die Steuereinheit (42) mit einem Zeitmessmittel für eine kontinuierliche Ermittlung des Zeitpunkts, zu dem das Gewebe, das sich in einem bestimmten Abstand von der Heizvorrichtung (10) befindet, eine bestimmte Temperatur erreicht hat, wirkverbunden ist.

6. Vorrichtung nach Anspruch 1 oder 2, **dadurch gekennzeichnet, dass** das Speichermittel (52) konstruiert ist, um Ableitungsdaten von einem bestimmten Körpergewebe zu speichern, die die Fähigkeit des Gewebes betreffen, Wärme abzuleiten.

7. Vorrichtung nach Anspruch 1 oder 2, **dadurch gekennzeichnet, dass** der erste Temperaturfühler (20) konstruiert ist, um in das Gewebe, dem Wärme zugeführt werden soll, einzudringen und eingeführtzu werden.

8. Vorrichtung nach Anspruch 1 oder 2, **dadurch gekennzeichnet, dass** der zweite Temperaturfühler (30) in der Nähe des Heizmittels (26) bereitgestellt ist, um die Temperatur in dem Gewebe in der Nähe des Katheters (10) füreine Behandlung zu messen.

9. Vorrichtung nach Anspruch 8, **dadurch gekennzeichnet, dass** der dritte Temperaturfühler (34) konstruiert ist, um in das Gewebe, dem Wärme zugeführt werden soll, in einem Abstand von dem Katheter (10) für die Behandlung einzudringen und eingeführtzu werden, der zwischen dem ersten (20) und dem zweiten (30) Temperaturfühler liegt.

10. Vorrichtung nach Anspruch 1 oder 2, **dadurch gekennzeichnet, dass** das Heizmittel (26) eine Mikrowellenantenne (26) zum Erwärmen des umgebenden Gewebes ist.

11. Vorrichtung nach Anspruch 3, **dadurch gekennzeichnet, dass** die Steuereinheit (42) mit einer Anzeigeeinheit (33) für eine Darstellung der Temperatur in unterschiedlichen Teilen des zu behandelnden Gewebes wirkverbunden ist, und dass die Steuereinheit (42) konfiguriert ist, um auf der Basis von Signalen des ersten Temperaturfühlers (20) und durch Berechnung auf der Basis, wie sich die Gewebetemperatur in dem Gewebe, in das der erste Temperaturwandler (20) eingesetzt ist, über die Zeit verändert, die darzustellende Temperatur zu bestimmen.

12. Vorrichtung nach einem der vorhergehenden Ansprüche, wobei die Steuereinheit (42) konfiguriert ist, um in der Lage zu sein, zwei unterschiedliche automatische Verfahren auszuführen, um zu ermitteln, wann die Energiezufuhrzu dem Heizmittel (26) beendet werden soll.

13. Vorrichtung nach Anspruch 12, wobei ein automatisches Verfahren zum Ermitteln, wann die Energiezufuhr zu dem Heizmittel (26) beendet werden soll, auf der Schätzung des Ausmaßes des Zelltods basierend auf der gemessenen Temperaturen und der Zeitdauer seit Beginn der Energiezufuhr zu dem Heizmittel (26) basiert.

14. Vorrichtung nach Anspruch 12, wobei ein automatisches Verfahren zum Ermitteln, wann die Energiezufuhr zu dem Heizmittel (26) beendet werden soll, auf einer Energiemenge basiert, die dem Heizmittel (26) seit Beginn der Energiezufuhrzu dem Heizmittel (26) zugeführt wird.

15. Vorrichtung nach Anspruch 12, wobei die beiden automatischen Verfahren zum Ermitteln, wann die Energiezufuhrzu dem Heizmittel (26) beendet werden soll, diejenigen nach Anspruch 13 beziehungsweise 14 sind.

16. Vorrichtung nach einem der Ansprüche 13, 14 oder 15, wobei die Steuereinheit (42) konfiguriert ist, um eine Auswahl zu treffen, welches der beiden unterschiedlichen automatischen Verfahren zum Ermitteln, wann die Energiezufuhr zu dem Heizmittel (26) beendet werden soll, verwendet werden soll.

17. Vorrichtung nach Anspruch 16, wobei die Steuereinheit (42) konfiguriert ist, um die Auswahl des Verfahrens basierend auf Unterschieden zwischen gleichzeitigen Temperaturwerten des ersten Fühlers (20) und des zweiten Fühlers (30), oder alternativ basierend auf Unterschieden zwischen gleichzeitigen Temperaturwerten des dritten Fühlers (34) und des zweiten Fühlers (30), oder basierend auf einer Kombination davon zu treffen.

18. Vorrichtung für die Zufuhr von Wärme zu Körpergewebe nach Anspruch 1, ferner umfassend, dass die Steuereinheit konfiguriert (42) ist, um das Ausmaß des zerstörten Gewebes mit Hilfe von Gleichung 1, unterstützt durch Gleichung 2 oder ähnliche Gleichungen, zu berechnen, und wobei Gleichung 1 lautet:

$$\rho c \frac{dT}{dt} = \lambda \Delta T - \varpi_b \rho_b c_b \rho (T - T_a) + Q_s + Q_m$$

wobei $\rho$ (rho) (kg m$^{-3}$) die Dichte der Prostata ist, c (J kg$^{-1}$ K$^{-1}$) die spezifische Wärmekapazität der Prostata ist, T (°C) die Temperatur der Prostatazu der Zeit t (Sekunden) ist, $\lambda$ (W m$^{-1}$ K$^{-1}$) die Wärmeleitung in der Prostata ist, $\Delta$ der Laplace-Operator ist, $\varpi_b$ die Perfusion des Gewebes (m$^3$ kg$^{-1}$ s$^{-1}$) ist, pb (kg) m$^{-3}$) die Dichte des Blutes ist, cb (J kg$^{-1}$ K$^{-1}$) die spezifische Wärmekapazität des Blutes ist, Ta die arterielle Temperatur (°C) ist, Qs (W m$^{-3}$) die Wärmeerzeugung als Ergebnis der Mikrowellenabsorption ist, und Qm (W m$^{-3}$) die Wärmeerzeugung durch den Metabolismus ist, wobei Gleichung 2 lautet:

$$\Omega = A \int e^{-E_a/(RT)} dt$$

wobei $\Omega$ (Omega) der Grad einer akkumulierten Schädigung während der Behandlungszeit t ist, A die Arrhenius-Konstante (3,110$^{98}$ s$^{-1}$) ist, $E_a$ die Aktivierungsenergie der Zellen (6,310$^5$ J mol$^{-1}$) ist, R (J mol$^{-1}$ K$^{-1}$) die universelle Gaskonstante ist, und T (K) die absolute Temperatur des Gewebes ist, wobei angenommen wird, dass das Gewebe bei $\Omega \geq 1$ zerstört wird.

19. Vorrichtung für die Zufuhr von Wärme zu Körpergewebe nach einem der vorhergehenden Ansprüche, wobei der Ausrichtungsmesser (23) die Ausrichtung des Katheters relativ zu einer absoluten Referenz erfasst..

20. Vorrichtung für die Zufuhr von Wärme zu Körpergewebe nach Anspruch 19, wobei der Ausrichtungsmesser (23) die Ausrichtung des Katheters relativ zu der Gravitationskraft erfasst.

21. Vorrichtung für die Zufuhr von Wärme zu Körpergewebe nach Anspruch 19 oder 20, wobei der Ausrichtungsmesser (23) als 3-Achsen-Beschleunigungsmesser und/oder 3-Achsen-Gyroskop enthalten ist.

22. Vorrichtung für die Zufuhr von Wärme zu Körpergewebe nach Anspruch 21, wobei der Ausrichtungsmesser (23) als 3-Achsen-Beschleunigungsmesser enthalten ist.

23. Vorrichtung für die Zufuhr von Wärme zu Körpergewebe nach Anspruch 21, wobei der Ausrichtungsmesser (23) als 3-Achsen-Gyroskop enthalten ist.

24. Vorrichtung für die Zufuhr von Wärme zu Körpergewebe nach einem der Ansprüche 19-23, wobei der Ausrichtungsmesser (23) unter Verwendung dünner Drähte, die sich durch den Katheter erstrecken, mit einem Indikator oder einer Anzeige verbunden ist.

25. Vorrichtung für die Zufuhr von Wärme zu Körpergewebe nach einem der Ansprüche 19-24, wobei der Ausrichtungsmesser (23) mit seiner Referenzrichtung Null oder einem bekannten Versatzwert relativ zu der Katheteröffnung für eine Sonde oder einen Träger (22) angeordnet ist.

26. Vorrichtung für die Zufuhr von Wärme zu Körpergewebe nach einem der Ansprüche 19-25, wobei der Ausrichtungsmesser (23) mit der Steuereinheit (42) wirkverbunden ist.

27. Vorrichtung für die Zufuhr von Wärme zu Körpergewebe nach Anspruch 26, wobei die Steuereinheit (42) konfiguriert ist, um ein Steuersignal zu berechnen und an die Energiezufuhreinheit (27) zu senden, die dem Heizmittel Energie zuführt, so dass das Heizmittel (26) beginnt, basierend auf Eingaben von dem Ausrichtungsmesser (23) Energie zum Erwärmen des Gewebes abzugeben.

**Revendications**

1. Dispositif de fourniture de chaleur au tissu de l'organisme, comprenant :

   - un cathéter (10) ayant une pointe (12) insérable dans une vessie humaine et soutenant un moyen de chauffage (26),
   - un support allongé (22) soutenant un premier transducteur de température (20), un deuxième transducteur de température (30) et un troisième transducteur de température (34) espacés l'un de l'autre, ledit support allongé (22) pouvant s'étendre du cathéter à proximité dudit moyen de chauffage (26) dans le tissu de l'organisme,
   - une unité de commande ;
   - un moyen de mémoire,

   dans lequel
   l'unité de commande (42) est connectée fonctionnellement au moyen de mémoire (52) pour stocker des données de chauffage correspondant à la survie de cellules en fonction de la température de lacellule et du temps, et est également connectée fonctionnellement auxdits premier transducteur de température (20), deuxième transducteur de température (30) et troisième transducteur de température (34),
   ladite unité de commande (42) est configurée pour obtenir un premier ensemble de données de température à partir desdits premier transducteur de température (20), deuxième transducteur de température (30) et troisième transducteur de température (34), et
   ladite unité de commande est configurée pour indiquer des différences entre des températures desdits premier, deuxième et/ou troisième transducteurs, **caractérisé en ce que** le dispositif comprend en outre un appareil de mesure d'orientation (23) destiné à détecter une orientation du cathéter (10).

2. Dispositif selon la revendication 1, **caractérisé en ce que** ladite unité de commande (42) est connectée fonctionnellement audit moyen de mémoire (52) pour le stockage d'un second ensemble de données de température correspondant àdes changements de température estimés dans le tissu de l'organisme à différentes distances dudit cathéter, et pour comparer ledit premier ensemble de données de température audit second ensemble de données de température.

3. Dispositif selon la revendication 1 ou 2, **caractérisé en ce que** les différences entre des valeurs de température simultanées du premier transducteur (20) et du deuxième transducteur (30) sont stockées et affichées.

4. Dispositif selon la revendication 3, **caractérisé en ce que** des différences entre des valeurs de température simultanées du troisième transducteur (20) et du deuxième transducteur (30) sont stockées et affichées.

5. Dispositif selon la revendication 1 ou 2, **caractérisé en ce que** ladite unité de commande (42) est connectée fonctionnellement à un moyen de mesure du temps pour la détermination continue du moment où le tissu situé à une certaine distance du dispositif de chauffage (10) a atteint une certaine température.

6. Dispositif selon la revendication 1 ou 2, **caractérisé en ce que** le moyen de mémoire (52) est conçu pour stocker des données de dissipation d'un certain tissu de l'organisme qui se rapportent à la capacité de dissipation de la chaleur du tissu.

7. Dispositif selon la revendication 1 ou 2, **caractérisé en ce que** ledit premier transducteur de température (20) est conçu pour pénétrer et être inséré dans le tissu devant être fourni en chaleur.

8. Dispositif selon la revendication 1 ou 2, **caractérisé en ce que** le deuxième transducteur de température (30) est prévu à proximité du moyen de chauffage (26) pour mesurer la température dans le tissu à proximité du cathéter (10) destiné au traitement.

9. Dispositif selon la revendication 8, **caractérisé en ce que** le troisième transducteur de température (34) est conçu pour pénétrer et être inséré dans le tissu devant être fourni en chaleur, à une distance dudit cathéter (10) destiné au traitement qui se situe entre le premier (20) et le deuxième (30) transducteur de température.

10. Dispositif selon la revendication 1 ou 2, **caractérisé en ce que** le moyen de chauffage (26) est une antenne à hyperfréquences (26) destinée à chauffer le tissu environnant.

**11.** Dispositif selon la revendication 3, **caractérisé**
**en ce que** l'unité de commande (42) est connectée fonctionnellement à une unité d'affichage (33) destinée à la présentation de la température dans différentes parties du tissu sous traitement, et
**en ce que** ladite unité de commande (42) est configurée pour déterminer la température à présenter sur la base de signaux provenant dudit premier transducteur de température (20) et par un calcul sur la base de la manière dont la température du tissu est modifiée au cours du temps dans le tissu dans lequel le premier transducteur de température (20) est inséré.

**12.** Dispositif selon l'une quelconque des revendications précédentes, dans lequel l'unité de commande (42) est configurée pour être capable d'exécuter deux procédés automatiques différents destinés à déterminer le moment auquel arrêter la fourniture en énergie du moyen de chauffage (26).

**13.** Dispositif selon la revendication 12, dans lequel un procédé automatique destiné à déterminer le moment auquel arrêter la fourniture en énergie du moyen de chauffage (26) est basé sur l'estimation de la quantité de mort cellulaire sur la base de températures mesurées et de la durée depuis le début de fourniture en énergie du moyen de chauffage (26).

**14.** Dispositif selon la revendication 12, dans lequel un procédé automatique destiné à déterminer le moment auquel arrêter la fourniture en énergie du moyen de chauffage (26) est basé sur une quantité d'énergie fournie au moyen de chauffage (26) depuis le début de fourniture en énergie du moyen de chauffage (26).

**15.** Dispositif selon la revendication 12, dans lequel les deux procédés automatiques destinés à déterminer le moment auquel arrêter la fourniture en énergie du moyen de chauffage (26) sont ceux des revendications 13 et 14 respectivement.

**16.** Dispositif selon l'une quelconque des revendications 13, 14 ou 15, dans lequel l'unité de commande (42) est configurée pour faire un choix parmi l'un des deux procédés automatiques différents à utiliser destinés à déterminer le moment auquel arrêter la fourniture en énergie du moyen de chauffage (26).

**17.** Dispositif selon la revendication 16, dans lequel l'unité de commande (42) est configurée pour faire le choix du procédé sur la base de différences entre des valeurs de température simultanées du premier transducteur (20) et du deuxième transducteur (30) ou, en variante, sur la base de différences entre des valeurs de température simultanées du troisième transducteur (34) et du deuxième transducteur (30), ou sur la base d'une combinaison de ceux-ci.

**18.** Dispositif de fourniture de chaleur au tissu de l'organisme selon la revendication 1, comprenant en outre la configuration de ladite unité de commande (42) pour calculer la quantité de tissu détruit à l'aide de l'éq. 1 appuyée par l'éq. 2 ou d'équations similaires, et où l'éq. 1 lit :

$$\rho c \frac{dT}{dt} = \lambda \Delta T - \varpi_b \rho_b c_b \rho (T - T_a) + Q_s + Q_m$$

où $\rho$ (rho) (kg m$^{-3}$) est la densité de la prostate, c (J kg$^{-1}$ K$^{-1}$) est la capacité calorifique spécifique de la prostate, T (°C) est la température de la prostate au temps t (secondes), $\lambda$ (W m$^{-1}$ K$^{-1}$) est la conduction thermique dans la prostate, $\Delta$ est l'opérateur de Laplace, $\varpi_b$ est la perfusion du tissu (m$^3$ kg$^{-1}$ s$^{-1}$), pb (kg m$^{-3}$) est la densité du sang, cb (J kg$^{-1}$ K$^{-1}$) est la capacité calorifique spécifique du sang, Ta est la température artérielle (°C), Qs (W m$^{-3}$) est la génération de la chaleur résultant de l'absorption des hyperfréquences, et Qm (W m$^{-3}$) est la génération de chaleur par métabolisme, et où l'éq. 2 lit :

$$\Omega = A \int e^{-E_a/(RT)} dt$$

où $\Omega$ (oméga) est le degré de dommage accumulé pendant le temps de traitement t, A est la constante d'Arrhenius (3,110$^{98}$ s$^{-1}$), E$_a$ est l'énergie d'activation des cellules, (6,310$^5$ J mol$^{-1}$), R (J mol$^{-1}$K$^{-1}$) est la constante de gaz universelle, et T (K) est la température absolue du tissu, le tissu étant supposé détruit à $\Omega \geq 1$.

**19.** Dispositif de fourniture de chaleur au tissu de l'organisme selon l'une quelconque des revendications précédentes, dans lequel le dispositif de mesure d'orientation (23) détecte l'orientation du cathéter par rapport à une référence absolue.

**20.** Dispositif de fourniture de chaleur au tissu de l'organisme selon la revendication 19, dans lequel le dispositif de mesure d'orientation (23) détecte l'orientation du cathéter par rapport à la force gravitationnelle.

**21.** Dispositif de fourniture de chaleur au tissu de l'organisme selon la revendication 19 ou 20, dans lequel le dispositif de mesure d'orientation (23) est réalisé sous la forme d'un accéléromètre à3 axes et/ou d'un gyroscope à 3 axes.

**22.** Dispositif de fourniture de chaleur au tissu de l'organisme selon la revendication 21, dans lequel le dispositif de mesure d'orientation (23) est réalisé sous la forme d'un accéléromètre à 3 axes.

**23.** Dispositif de fourniture de chaleur au tissu de l'organisme selon la revendication 21, dans lequel le dispositif de mesure d'orientation (23) est réalisé sous la forme d'un gyroscope à 3 axes.

**24.** Dispositif de fourniture de chaleur au tissu de l'organisme selon l'une quelconque des revendications 19 à 23, dans lequel le dispositif de mesure d'orientation (23) est connecté à un indicateur ou à un affichage à l'aide de fils minces s'étendant à travers le cathéter.

**25.** Dispositif de fourniture de chaleur au tissu de l'organisme selon l'une quelconque des revendications 19 à 24, dans lequel le dispositif de mesure d'orientation (23) est agencé avec sa direction de référence étant zéro ou une valeur de décalage connue par rapport à l'ouverture dans le cathéter pour la sonde ou le support (22).

**26.** Dispositif de fourniture de chaleur au tissu de l'organisme selon l'une quelconque des revendications 19 à 25, dans lequel le dispositif de mesure d'orientation (23) est connecté fonctionnellement à ladite unité de commande (42).

**27.** Dispositif de fourniture de chaleur au tissu de l'organisme selon la revendication 26, dans lequel l'unité de commande (42) est configurée pour calculer et envoyer un signal de commande à l'unité de fourniture en énergie (27), qui fournit de l'énergie au moyen de chauffage de telle sorte que le moyen de chauffage (26) commence à émettre de l'énergie pour chauffer le tissu, sur la base d'entrées dudit dispositif de mesure d'orientation (23).

Fig. 1

Fig. 2

Fig. 3

Fig. 4a

Fig. 4b

Fig. 4c

*Fig. 5*

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0370890 A
- WO 9636288 A
- SE 9600649 W
- US 6445957 B1

**Non-patent literature cited in the description**

- *British Journal of Urology,* 1996, vol. 78, 564-572